# FASCICULE DE BREVET EUROPEEN

(11) **EP 0 627 919 B1**
(45) Date de publication et mention de la délivrance du brevet: **12.03.1997**
(21) Numéro de dépôt: 92923852.5
(22) Date de dépôt: 22.10.1992
(51) Int. Cl.: A61K 31/425

(54) **APPLICATION DE L'AMINO-2 TRIFLUOROMETHOXY-6 BENZOTHIAZOLE (RILUZOLE) POUR OBTENIR UN MEDICAMENT DESTINE AU TRAITEMENT DES MALADIES DU MOTONEURONE**
VERWENDUNG VON AMINO-2-TRIFLUORMETHOXY-6-BENZOTHIAZOL (RILUZOL) ZUR HERSTELLUNG EINES MEDIKAMENTES ZUR BEHANDLUNG VON ERKRANKUNGEN DES MOTONEURONS
APPLICATION OF 2-AMINO 6-TRIFLUOROMETHOXY BENZOTHIAZOLE (RILUZOLE) FOR OBTAINING A DRUG USEFUL IN THE TREATMENT OF MOTOR NEURON DISEASES

(30) Priorité: 06.03.1992 FR 9202696
(43) Date de publication de la demande: 14.12.1994
(73) Titulaire: RHONE-POULENC RORER S.A., 92160 Antony (FR)
(72) Inventeur: LOUVEL, Erik, F-04100 Manosque (FR)
(74) Mandataire: Savina, Jacques
(86) Numéro de dépôt international: FR9200992
(87) Numéro de publication internationale: WO9317683

(56) Documents cités:
- EP-A- 0 050 551
- EP-A- 0 282 971
- EP-A- 0 374 041
- WO-A-91/17984
- WO-A-91/18892
- THE JOURNAL OF NEUROSCIENCE, vol. 9, no. 11, Novembre 1989, pages 3720-3727; C. MALGOURIS ET AL.: 'RILUZOLE, A NOVEL ANTIGLUTAMATE, PREVENTS MEMORY LOSS AND HIPPOCAMPAL NEURONAL DAMAGE IN ISCHEMIC GERBILS'
- BRITISH JOURNAL OF PHARMACOLOGY, vol. 97, page 583P; D. GIRDLESTONE ET AL.: 'RILUZOLE ANTAGONISES EXCITATORY AMINO ACID-EVOKED FIRING IN RAT FACIAL MOTONEURONES IN VIVO'
- THERAPIE, vol. 45, no. 3, 1990, pages 277-279; TH.L. MUNSAT ET AL.: 'EXCITOTOXINS AND AMYOTROPHIC LATERAL SCLEROSIS'
- ANN NEUROL, vol. 22, no. 5, 1987, pages 575-579; A. PLAITAKIS ET AL.: 'ABNORMAL GLUTAMATE METABOLISM IN AMYOTROPHIC LATERAL SCLEROSIS'
- NEUROLOGY, vol. 41, no. 3, 1991, pages 392-393; A. PLAITAKIS ET AL.: 'DYSREGULATION OF GLUTAMATE METABOLISM IN ALS: CORRELATION WITH GENDER AND DISEASE TYPE'
- 'PSCHYREMBEL KLINISCHES W RTERBUCH', 1986, WALTER DE GRUYTER, BERLIN, NEW YORK, p. 67: "Amyotrophische Lateralsklerose"
- 'THE MERCK MANUAL', 1987, MERCK & CO., INC., RAHWAY, N.J., pp. 1438-1439: "MOTOR NEURON DISEASES"

## Description

La présente invention concerne l'application de l'amino-2 trifluorométhoxy-6 benzothiazole ou un sel de ce composé avec un acide pharmaceutiquement acceptable pour obtenir un médicament destiné au traitement des maladies du motoneurone, notamment de la sclérose latérale amyotrophique et, en particulier, la sclérose latérale amyotrophique à début bulbaire ou à forme bulbaire.

Il est connu que l'amino-2 trifluorométhoxy-6 benzothiazole (dénomination commune internationale : riluzole) est utile comme médicament anticonvulsivant, anxiolytique et hypnotique (brevet EP 50551), dans le traitement de la schizophrénie (EP 305276), dans le traitement des troubles du sommeil et de la dépression (EP 305277), dans le traitement des désordres cérébrovasculaires et comme anesthésique (EP 282971).

EP-A-374041 décrit des dérivés de la benzothiazolamine-2 à l'exception du riluzole et leur utilité dans le traitement de la sclérose latérale amyotrophique.

Il a maintenant été trouvé que l'amino-2 trifluorométhoxy-6 benzothiazole ou un sel ce composé avec un acide pharmaceutiquement acceptable est utile dans le traitement des maladies du motoneurone, notamment de la sclérose latérale amyotrophique et, en particulier, la sclérose latérale amyotrophique à début bulbaire ou à forme bulbaire.

Cette application a été déterminée chez l'homme dans une étude en double aveugle contre placebo : 77 patients atteints de maladies motoneurones et notamment de sclérose latérale amyotrophique ont été traités par 2x50 mg/jour de riluzole par voie orale (comprimés dosés à 50 mg) pendant une période de 12 à 18 mois et 78 patients ont reçu un placebo.

Les résultats obtenus sont analysés en terme de survie dans l'étude étant entendu que, les "morts de l'étude" (sortie de l'étude) comprennent les personnes réellement mortes mais également les personnes dont l'état clinique nécessite une trachéotomie ou le, passage sous assistance respiratoire.

Dans cette étude, 51 % des patients sous placebo sont morts alors que ce pourcentage descend à 44 % chez les patients sous riluzole (la probabilité dans le test de Wilcoxon (R. L. PREUCTICE, Biometrika, 65, 167-179 (1978)) est égale à 0,018 et la probabilité dans le test de Logrank stratifié (R. PETO et J. PETO, Journal of the Royal Statistical Society, series A, vol. 135, 185-207 (1972)) est égale à 0,06).

Chez les sujets atteints de sclérose latérale amyotrophique à début bulbaire ou à forme bulbaire (forme la plus grave de la maladie; la survie moyenne habituelle de ce type de patients est inférieure à 3 ans), 65 % des patients sous placebo sont morts alors que ce pourcentage descend à 47 % chez les patients sous riluzole (la probabilité dans le test de Wilcoxon est égale à 0,011 et la probabilité dans le test de Logrank (R. PETO et J. PETO, Journal of the Royal Statistical Society, series A, vol. 135, 185-207 (1972)) est égale à 0,032).

L'amino-2 trifluorométhoxy-6 benzothiazole augmente donc de manière statistiquement significative la survie des patients atteints de maladies du motoneurone et notamment de sclérose latérale amyotrophique et cet effet est particulièrement net chez les patients atteints de sclérose latérale amyotrophique à début bulbaire ou à forme bulbaire.

L'amino-2 trifluorométhoxy-6 benzothiazole peut être préparé selon le procédé décrit dans le brevet EP 50551.

Comme sels pharmaceutiquement acceptables peuvent être notamment cités les sels d'addition avec les acides minéraux tels que chlorhydrate, sulfate, nitrate, phosphate ou organiques tels que acétate, propionate, succinate, oxalate, benzoate, fumarate, maléate, méthanesulfonate, iséthionate, théophilline-acétate, salicylate, phénolphtalinate, méthylène-bis-β-oxynaphtoate ou des dérivés de substitution de ces dérivés.

Les médicaments selon l'invention sont constitués par l'amino-2 trifluorométhoxy-6 benzothiazole sous forme libre ou sous forme d'un sel d'addition avec un acide pharmaceutiquement acceptable, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semisynthétiques ou des polyéthylèneglycols.

Les compositions pour administration topique peuvent être par exemple des crèmes, lotions, collutoires, gouttes nasales ou aérosols.

Les doses dépendent de l'effet recherché, de la durée du traitement et de la voie d'administration utilisée ; elles sont généralement comprises entre 50 et 400 mg par jour par voie orale pour un adulte avec des doses unitaires allant de 25 à 200 mg de substance active.

D'une façon générale, le médecin déterminera la posologie appropriée en fonction de l'âge, du poids et de tous les autres facteurs propres au sujet à traiter.
Les exemples suivants illustrent des médicaments selon l'invention :

### Exemple A

On prépare, selon la technique habituelle, des comprimés dosés à 50 mg de produit actif ayant la composition suivante :

| | |
|---|---|
| - Amino-2 trifluorométhoxy-6 benzothiazole | 50 mg |
| - Mannitol | 64 mg |
| - Cellulose microcristalline | 50 mg |
| - Polyvidone excipient | 12 mg |
| - Carboxyméthylamidon sodique | 16 mg |
| - Talc | 4 mg |
| - Stéarate de magnésium | 2 mg |
| - Silice colloïdale anhydre | 2 mg |
| - Mélange de méthylhydroxypropylcellulose, polyéthylèneglycol 6000, dioxyde de titane (72-3,5-24,5) | |
| q.s.p. 1 comprimé pelliculé terminé à 245 mg | |

### Exemple B

On prépare, selon la technique habituelle, des gélules dosées à 50 mg de produit actif ayant la composition suivante :

| | |
|---|---|
| - Amino-2 trifluorométhoxy-6 benzothiazole | 50 mg |
| - Cellulose | 18 mg |
| - Lactose | 55 mg |
| - Silice colloïdale | 1 mg |
| - Carboxyméthylamidon sodique | 10 mg |
| -Talc | 10 mg |
| - Stéarate de magnésium | 1 mg |

### Exemple C

On prépare une solution injectable contenant 10 mg de produit actif ayant la composition suivante :

| | |
|---|---|
| - Amino-2 trifluorométhoxy-6 benzothiazole | 10 mg |
| - Acide benzoïque | 80 mg |
| - Alcool benzylique | 0,06 cm3 |
| - Benzoate de sodium | 80 mg |
| - Ethanol à 95 % | 0,4 cm3 |
| - Hydroxyde de sodium | 24 mg |
| - Propylène glycol | 1,6 cm3 |
| - Eau q.s.p. | 4 cm3 |

## Revendications

1. Application de l'amino-2 trifluorométhoxy-6 benzothiazole ou un sel de ce composé avec un acide pharmaceutiquement acceptable pour obtenir un médicament destiné au traitement des maladies du motoneurone.

2. Application selon la revendication 1 de l'amino-2 trifluorométhoxy-6 benzothiazole ou un sel de ce composé avec un acide pharmaceutiquement acceptable pour obtenir un médicament destiné au traitement de la sclérose latérale amyotrophique.

3. Application selon la revendication 2 pour obtenir un médicament destiné au traitement de la slérose latérale amyotrophique à début bulbaire.

4. Application selon la revendication 2 pour obtenir un médicament destiné au traitement de la slérose latérale amyotrophique à forme bulbaire.

5. Application selon l'un des revendications 1 à 4 pour obtenir un médicament comprenant 25 à 200 mg d'amino-2 trifluorométhoxy-6 benzothiazole.

## Claims

1. Use of 2-amino-6-(trifluoromethoxy)benzothiazole, or a salt of this compound with a pharmaceutically acceptable acid, in the manufacture of a medicament for use in the treatment of motor neuron diseases.

2. Use according to Claim 1 of 2-amino-6-(trifluoromethoxy)benzothiazole, or a salt of this compound with a pharmaceutically acceptable acid, in the manufacture of a medicament for use in the treatment of amyotrophic lateral sclerosis.

3. Use according to Claim 2 in the manufacture of a medicament for use in the treatment of amyotrophic lateral sclerosis with early bulbar involvement.

4. Use according to Claim 2 in the manufacture of a medicament for use in the treatment of amyotrophic lateral sclerosis, bulbar form.

5. Use according to one of Claims 1 to 4 in the manufacture of a medicament comprising 25 to 200 mg of 2-amino-6-(trifluoromethoxy)benzothiazole.

## Patentansprüche

1. Verwendung von 2-Amino-6-trifluormethoxy-benzothiazol oder einem Salz dieser Verbindung mit einer pharmazeutisch akzeptablen Säure zur Herstellung eines Arzneimittels für die Behandlung von Erkrankungen des Motoneurons.

2. Verwendung nach Anspruch 1 von 2-Amino-6-trifluormethoxy-benzothiazol oder einem Salz dieser Verbindung mit einer pharmazeutisch akzeptablen Säure zur Herstellung eines Arzneimittels für die Behandlung von amyotrophischer Lateralsklerose.

3. Verwendung nach Anspruch 2 zur Herstellung eines Arzneimittels für die Behandlung von amyotrophischer Lateralsklerose mit bulbärem Beginn.

4. Verwendung nach Anspruch 2 zur Herstellung eines Arzneimittels für die Behandlung von amyotrophischer Lateralsklerose in bulbärer Form.

5. Verwendung nach Anspruch 1 bis 4 zur Herstellung eines Arzneimittels, das 25 mg bis 200 mg 2-Amino-6-trifluormethoxy-benzothiazol umfaßt.
